# FASCICULE DE BREVET EUROPEEN

(11) **EP 1 773 450 B1**
(45) Date de publication et mention de la délivrance du brevet: **16.02.2011**
(21) Numéro de dépôt: 05763958.5
(22) Date de dépôt: 13.07.2005
(51) Int. Cl.: A61P 9/10, A61K 45/06, A61K 9/22

(54) **COMBINAISONS THERAPEUTIQUES**
THERAPEUTISCHE KOMBINATIONEN
THERAPEUTICALLY COMBINATIONS

(30) Priorité: 22.07.2004 BE 200400364
(43) Date de publication de la demande: 18.04.2007
(73) Titulaire: Messadek, Jallal, 4000 Liège (BE)
(72) Inventeur: Messadek, Jallal, 4000 Liège (BE)
(86) Numéro de dépôt international: PCT/BE2005/000112
(87) Numéro de publication internationale: WO 2006/007671

(56) Documents cités:
- WO-A-02/062322
- WO-A-2004/049095
- WO-A-2005/011642
- DE-A1- 19 910 682
- US-B1- 6 235 311
- MCCARTY M F: "Co-administration of equimolar doses of betaine may alleviate the hepatotoxic risk associated with niacin therapy" MEDICAL HYPOTHESES, vol. 55, no. 3, septembre 2000 (2000-09), pages 189-194, XP009059259 ISSN: 0306-9877 cité dans la demande
- ,
- MUNOZ A ET AL: "MICRONISED FENOFIBRATE" ATHEROSCLEROSIS, ELSEVIER IRELAND LTD, IE, vol. 110, no. SUPPL, 1 January 1994 (1994-01-01), pages S45-S48, XP009023822 ISSN: 0021-9150
- MUNOZ A ET AL: "MICRONISED FENOFIBRATE" 1 January 1994 (1994-01-01), ATHEROSCLEROSIS, ELSEVIER IRELAND LTD, IE, PAGE(S) S45 - S48 , XP009023822 ISSN: 0021-9150

## Description

### But de l'invention

Le but de la présente invention est une composition pharmaceutique comprenant une bétaine comicronisé avec du fénofibrate. L'association et la co-administration d'au moins une bétaine permettant de réduire les effets secondaires liés à l'administration des agents anti-cholestérol comme la fénofibrate notamment leurs effets délétères sur le foie, le pancréas et les reins. L'administration des agents anti-cholestérol induit également l'élévation des enzymes du foie ou transaminases et/ou l'élévation des taux d'homocystéine dans le corps, que l'association et la co-administration d'au moins une bétaine permet de réduire. De par ses propriétés cardiovasculaires, anti-thrombotiques, anti-agrégantes, anti-adhésives, lipotropes ainsi que son activité sur la pression diastolique, l'association et la co-administration d'au moins une bétaine avec un agent anti-cholestérol permet d'améliorer l'efficacité thérapeutique de ces agents anti-cholestérol tout en réduisant leurs effets secondaires. Une telle combinaison serait particulièrement avantageuse par rapport aux traitements existants quant à la protection, sécurité et commodité d'emploi accrus qu'elle procure.

La composition de l'invention pourra être utilisée pour traiter et/ou prévenir les maladies cardiovasculaires, les maladies cardiovasculaires occlusives, l'hypertension vasculaire, les troubles du métabolisme lipidique, l'hyperlipidémie, les troubles liés aux cholestérols, les troubles liés aux triglycérides, les troubles liés aux lipoprotéines, les diabètes, le diabète de type 2, les maladies métaboliques, l'athérosclérose, la résistance à l'insuline, le syndrome de la résistance à l'insuline, le syndrome X et le syndrome métabolique.

### Etat de la technique

Les publications "Westphal et al, Effects of fenofibrate and gemfibrozil on plasma homocysteine Lancet. 2001 Jul 7;358 (9275):39-40" et "Gotto et al, Risks and benefits of continued aggressive statin therapy Clin Cardiol. 2003 Apr;26(4 Suppl 3):III3-12" montrent que l'administration de substances anti-cholestérol induit une élévation des transaminases ou une élévation des taux d'homocystéine. Un taux élevé d'homocystéine est un facteur reconnu de maladies vasculaires.

Les publications "Ethanol-induced hepatotoxicity and protective effect of betaine. Kanbak et al Cell Biochem Funct 2001 Dec;19(4):281-5" et "Betaine, ethanol, and the liver: a review. Barak. Et al, Alcohol 1996 Jul-Aug;13(4): 395-8" montrent que l'effet hépato-protecteur de la bétaine dans des modèles de stéatose expérimentale sur animaux n'est atteint qu'à des doses orales très élevées (0,5 % de bétaine/ unité de poids du corps, ce qui équivaut à 5 grammes/kilo de poids vif ou encore 350 g pour un homme de 70 kg) mais qui pourraient correspondre à la sévérité du challenge pathologique utilisé.

Le syndrome métabolique est défini selon Lindblad et al dans la publication "Metabolic syndrome and ischemic heart disease in elderly men and women. Am J Epidemiol 2001;153:481-9" comme la prévalence d'au moins 3 des symptômes suivants: obésité, hypertension, taux de triglycérides élevés, taux de cholestérol HDL bas, taux de glucose à jeun élevé.

L'usage oral de la bétaine pour réduire les taux d'homocystéine chez l'humain est connu depuis 3 décennies et abondamment documenté.

DE 19910682 enseigne l'association de la bétaine avec des fibrates. M.F. McCarty dans Medical Hypotheses, (2000) 55(3), 189-194 décrit l'association d'une bétaine avec une niacine dans un ratio équimolaire afin d'éviter les effets hépatotoxiques des niacines. Là bétaine n'est pas sous une forme à libération contrôlée.

On connaît de par WO 00/ 51596 de l'inventeur l'activité antithrombotique de la bétaine.

La publication "Betaine supplementation decreases plasma homocysteine concentrations but does not affect body weight, body composition, or resting energy expenditure in human subjects Ursula Schwab et al , Am J Clin Nutr 2002;76:961" décrit l'effet de la bétaine, chez des volontaires obèses, sur la pression diastolique, les taux d'homocysteine et les taux d'HDL.
La publication "Betaine, a Promising New Agent for Patients With Nonalcoholic Steatohepatitis: Results of a Pilot Study Manal F. Abdelmalek et al, The American Journal Of Gastroenterology Vol. 96, No. 9, 2001" décrit l'effet hépatoprotecteur de la bétaine et son effet régulateur sur les transaminases.

On connaît par WO 02/ 062322 de l'inventeur les formes à libérations contrôlées de bétaine ainsi que l'association de la bétaine avec les statines et le ciprofibrate. Ce document n'enseigne pas la combinaison d'une forme à libération contrôlée de bétaine avec une forme contenant des statines ou du ciprofibrate.

WO 02/ 062322 décrit l'association de la bétaine dans sa forme à libération immédiate avec les statines et le ciprofibrate mais ne décrit pas une composition pharmaceutique comprenant une bétaine sous une forme à libération contrôlée et/ou retard et/ou flottante combinée à un agent anti-cholestérol.

Aucune des publications précédentes ne décrit une composition pharmaceutique comprenant une bétaine sous une forme à libération contrôlée et/ou retard et/ou flottante combinée à un agent anti-cholestérol.

L'administration de la bétaine permet d'abaisser la concentration d'homocystéine dans le sang. Les patients homocystinuriques pour près de la moitié sont traités avec de hautes doses orales de bétaine de 6 grammes à 20 grammes par jour. Ces doses élevées de bétaine sont nécessaires afin d'atteindre des concentrations plasmatiques chez les patients de l'ordre de 200 à 400 µMol/L et par conséquent de réduire les taux d'homocystéine (An indirect response model of homocysteine suppression by betaine: optimising the dosage regimen of betaine in homocystinuria, Angela Matthews et al, Br J Clin Pharmacol, 54, 140-146). De telles doses sont inacceptables d'un point de vue « compliance » pour un patient.
Il ressort également qu'après administration orale du Cystadane® (poudre de bétaine anhydre) la biodisponibilité absolue de la bétaine apparaît comme étant très faible, de l'ordre de 10 % (Schwahn BC et al : Pharmacokinetics of oral betaine in healthy subjects and patients with homocystinuria. Br J Clin Pharm. 2003 Jan;55(1):6-13). Dans cette même publication, il apparaît que la bétaine après administration atteint rapidement un pic plasmatique (efficacité optimale) dont la durée est relativement courte.
Dans l'étude "Betaine, a Promising New Agent for Patients With Nonalcoholic Steatohepatitis: Results of a Pilot Study. Abdelmalek et al", la bétaine est administrée à des doses orales de 20 grammes jour, c'est-à-dire que pour atteindre une dose thérapeutiquement effective dans ce type de pathologie, similaire à celles induites par les agents anti-cholestérol, il est nécessaire d'administrer de grandes quantités de bétaine. Ces hautes doses étant en concordance avec celles utilisées dans les expérimentations animales.

Il apparaît donc que pour pouvoir atteindre un seuil de protection hépatique utile ou une réduction suffisante de l'homocystéine dans le corps, il est nécessaire d'administrer, en combinaison avec les agents anti-cholestérol, de grandes quantités de bétaine au détriment de la commodité du traitement, ce qui pour des traitements au long cours peut s'avérer contraignant et compromettre la stricte observance du patient et de là le résultat thérapeutique final.

Pour les raisons ci-dessus l'administration équimolaire de niacines avec la bétaine pourrait également souffrir de ce manque de commodité eu égard à la quantité thérapeutique des niacines à administrer, c'est-à-dire 4 à 8 grammes par jour en trois prises, dose à laquelle il faudrait ajouter la même quantité de bétaine, ce qui équivaudrait à une combinaison médicamenteuse de 8 à 16 grammes par jour. De plus, à la lumière des doses utilisées dans l'étude de Abdelmalek et al, il n'est pas sûr que cette dose équimolaire soit suffisante pour qu'un effet hépatoprotecteur de la bétaine puisse être atteint.

Pour les mêmes raisons de commodité, l'utilisation de niacines retard à la dose de 1000 à 2000 mg/jour serait encore contraignant (dose de 2 à 4 grammes de la combinaison niacin retard/bétaine à libération immédiate) ne procurerait qu'une faible teneur de 100 à 200 mg de bétaine totale circulante, ce qui est insuffisant pour l'effet protecteur recherché.

Concernant l'association d'une bétaine avec un fibrate les auteurs de DE 19910682 décrivent l'utilisation jusqu'à 20 grammes/jour d'une bétaine à libération immédiate. Dans l'exemple 3 il décrivent une tablette effervescente contenant 2 grammes de bétaine (à libération immédiate) plus 1,2 grammes de Gemfibrozil, plus les excipients permettant l'effervescence, ce qui selon l'état de la technique galénique devrait donner une tablette effervescente d'un poids total de ± 4 à 5 grammes et d'un diamètre de plus de 2 centimètres. Un tel médicament ne serait pas très commode pour une utilisation au long terme, sa taille et sa présentation risquant de compromettre une stricte observance de son usage. De plus la quantité de bétaine utilisée risque de ne pas procurer la protection désirée tant au niveau du foie que dans la réduction de l'homocystéine.

Concernant la combinaison d'une bétaine avec les statines dans WO 02/ 062322, l'inventeur décrit une combinaison où la bétaine est sous une forme à libération immédiate, c'est-à-dire que pour atteindre un effet protecteur il est nécessaire d'administrer plusieurs grammes par jour. Les statines sont administrées à de très faibles doses (de 0,2 mg à 80 mg par jour) et une combinaison avec une bétaine à libération immédiate en quantité suffisante pour un effet hépatoprotecteur et/ou thérapeutique (c'est-à-dire plusieurs grammes) pourrait nuire à l'absorption optimale de ces statines du fait de leur très faible ratio dans ladite combinaison. De fait, dans une telle combinaison les statines se retrouveraient noyées et entraînées par l'excès de bétaine, ceci en réduisant la quantité de statines qui atteindrait le flux sanguin compromettrait leur efficacité thérapeutique. Une combinaison dans cette forme, la bétaine étant un surfactant, aurait le désavantage de lessiver ou de masquer la quantité thérapeutique très minime des statines administrées, d'où la nécessité d'une administration particulière de la bétaine afin d'éviter de perdre ou de masquer la substance anti-cholestérol.

Il apparaît donc que les combinaisons de bétaine à libération immédiate avec des agents anti-cholestérol antérieurement décrites présentent de nombreux désavantages. Ces désavantages sont notamment dus aux interactions possibles des bétaines avec les agents anti-cholestérol sur les sites d'absorption, aux grandes quantités de bétaine à administrer avant d'atteindre des concentrations à effet thérapeutique protecteur et qui ne semblent pas atteintes dans ces combinaisons antérieures, et au manque de contrôle de la présence et de la concentration dans le corps des bétaines. De plus, les combinaisons connues antérieurement décrivent des formes galéniques contraignantes et manquant de commodité à l'usage ce qui est un facteur avéré de mauvaise observance dans les traitements au long cours et qui pourrait compromettre le résultat thérapeutique final.

De manière surprenante, l'inventeur a découvert que le fait de combiner une bétaine sous une forme à libération contrôlée et/ou retard et/ou flottante à un agent anti-cholestérol permettait d'éviter ces désavantages. Les combinaisons de l'invention améliorent la commodité d'usage et l'observance des traitements, sont plus sûres et plus efficaces, et ce résultat étant atteint tout en réduisant la quantité de bétaine, voire d'agent anti-cholestérol à administrer. En contrôlant de manière optimale la présence et la concentration de bétaine dans le corps, les combinaisons de l'invention, eu égard aux nombreuses propriétés pharmacologiques de la bétaine, sont plus efficaces et plus sûres thérapeutiquement comparativement aux combinaisons antérieures ainsi qu'aux traitements existants. L'utilisation des formes à libérations contrôlées et/ou retard et/ou flottantes de bétaine dans les combinaisons de l'invention constitue, par les avantages qu'elle apporte, une amélioration très notable à l'état de la technique.

### Manières de réaliser l'invention

Pour les fibrates on peut adopter les techniques de micronisation où au moins une bétaine est utilisée comme tensioactif ou surfactant, ceci permettant d'éviter l'emploi d'autres surfactants et les effets secondaires liés auxdits surfactants. L'invention a donc également pour objet un procédé de préparation de fibrate micronisé (taille moyenne en poids de moins de 10µm, en particulier de moins de 5µm, de préférence de moins de 2µm, voire de moins de 1µm), dans lequel du fibrate solide est micronisé en présence de bétaine, le rapport en poids fibrate /betaine étant avantageusement inférieur à 1.

La bétaine pourra être utilisée comme surfactant notamment dans les formes micronisées, micro et/ou nano sphéroïdiques et/ou sphéronisées de fénofibrate. La bétaine en tant que surfactant pourra être complètement ou en partie sous une forme à libération immédiate et/ou complètement ou en partie sous une forme à relargages et/ou libérations contrôlés.
L'avantage de l'utilisation d'une bétaine en tant surfactant ou tensioactif dans les combinaisons de l'invention réside dans le fait que la bétaine est très peu toxique, contrairement à d'autres surfactants utilisés avec des agents anti-cholestérol notamment le sodium lauryl sulfate utilisé dans les formulations de fenofibrate micronisé, notamment dans le Tricor ®. La bétaine dans ce cas pouvant jouer le multiple rôle de surfactant ou tensioactif, de substance thérapeutique réduisant les effets secondaires des agents anti-cholestérol et de substance thérapeutique protectrice quant à diverses pathologies notamment les maladies cardiovasculaires et le syndrome métabolique. La bétaine sert dans ce cas également à améliorer la biodisponibilité du fénofibrate par rapport à la biodisponibilité dudit agent sans surfactant ou tensioactif.
La composition suivant l'invention est donc exemple de tensioactif du type sodium lauryl sulfate. Selon une forme particulière, la composition contient en tant que surfactant ou tensioactif, sensiblement uniquement de la bétaine, en particulier de la glycine bétaine. La bétaine, en particulier la glycine bétaine, forme plus de 90% en poids, de préférence plus de 95% en poids, plus spécifiquement plus de 99% en poids des surfactants ou tensioactifs présents dans la composition.

La bétaine, en particulier la glycine bétaine, de préférence sous forme anhydre, en tant que surfactant ou tensioactif pourra être mélangée au fénofibrate avant, pendant ou après micronisation et/ou nanonisation (mise sous forme de particules inférieures au micron). La bétaine, en particulier la glycine bétaine, permet d'améliorer la biodisponibilité du fénofibrate lorsque le fénofibrate est micronisé ou nanonisé en présence de bétaine, en particulier de glycine bétaine. Dans un aspect particulier, ce mélange pourra comprendre additionnellement des excipients, notamment des excipients destinés à assurer et/ou contrôler une meilleure biodisponibilité, profil de libération, temps de résidence gastrique ou absorption par le corps, lesdits excipients pouvant être mélangés avant, pendant ou après micronisation.

Le terme bétaine et/ou au moins une bétaine et/ou bétaines dans le champ de la présente invention fait référence aux bétaines décrites et revendiquées dans les applications WO 00/ 51596, WO 02/ 062322 et PCT/ IB 02/ 04923 de l'inventeur.

Les termes " bétaine" et/ou " au moins une bétaine" et/ou "bétaines" employés dans la présente invention se réfèrent aux composés choisis dans les groupes consistant en des bétaines lipidiques, des lipides de bétaine, et/ou des bétaines de formule (CH₃)₃N⁺- (CH₂)ₙ -COO avec n un nombre entier de 1 à 5, (préférablement la glycine bétaine n = 1), leurs sels pharmaceutiquement acceptables, leurs esters, leurs précurseurs et leurs mélanges.
Les termes " bétaines lipidiques " et " lipides de bétaine " se réfèrent aux lipides de bétaine et/ou bétaines lipidiques qui sont des éléments structuraux des membranes que l'on trouve communément dans les fougères, les mousses, les mycétes, les champignons, les amibes, les eucaryotes, les plantes sauvages et les algues. Les lipides de bétaine sont des glycérolipides non phosphoriques éthers liés, qui ressemblent par leur structure générale à la plus communément connue phosphatidylcholine. Les plus communs des glycérolipides contiennent une portion de diacyl glycérol à laquelle un groupe polaire est attaché. Ce groupe polaire peut être une portion carbohydrate comme dans les galactolipides très abondants dans les plantes ou un phosphorylester comme dans les glycéro phospholipides, la classe lipidique la plus commune chez les animaux. Les lipides de bétaine représentent une troisième classe de glycérolipides dans lequel un alcool amine quaternaire est lié dans une liaison éther à la portion diacylglycerol. Ils peuvent être obtenus par extraction, biosynthèse ou par synthèse. Les lipides de bétaines diacylglyceryl- O-4' - (N, N, N-trimethyl) homoserine et une proche isoforme le diacylglyceryl - O-2' - (hydroxymethyl) (N,N,N- trimethyl)-β-alanine sont les plus communs.

Des aspects et particularités de l'invention sont décrits dans les revendications.

L'invention a donc pour objet une composition pharmaceutique à visage oral comprenant du fénofibrate comicronisé ou co nanonisé avec de la bétaine, en particulier de la glycine bétaine.

L'invention a encore pour objet une composition de fénofibrate contenant du fénofibrate comicronisé ou réduit en particules de moins de 1µm (nanonisé), en présence de bétaine, en particulier de glycine betaine ou d'un sel de celle-ci. De préférence au moins 50% du fénofibrate de la composition est comicronisé ou réduit en particules de moins de 1µm en présence de bétaine.

Les examples 1, 2 et 4-7 ne fait pas partie de l'invention.

### Exemple 1 comprimé betaine fibrate

| Ingrédient | mg/ comprimé |
|---|---|
| Betaine Anhydre | 450,00 |
| Fenofibrate micronisé | 100,00 |
| (5 à 20 µm) | |
| Lactose Ph. Eur. | 48,00 |
| Ethylcellulose | 110,00 |
| (Relargage .RTM.) | |
| Eau pure Ph. Eur. | 150,00* |
| Alcool Cetostearyl | 42,00 |
| Ph. Eur. | |
| Magnesium Stearate | 20,00 |
| Ph. Eur. | |
| Talc Ph. Eur. | 30,00 |
| TOTAL | 800,00 |

| | |
|---|---|
| * Eliminée durant le processus | |

### Procédé d'obtention

La bétaine, préférablement de la glycine betaine anhydre et le lactose ont été égrenés, puis transférés dans un granulateur vibrant et vaporisés avec de l'ethylcellulose et de l'eau. Les grains obtenus sont alors séchés à 60 degrés C. et passés par un tamis de 0,4 mm. Les granules obtenus sont alors mélangés avec l'alcool cetostearyl et le tout mixé énergiquement. La mixture obtenue est passée par un tamis de 0,5, laissée refroidir, puis est mixée avec le fenofibrate micronisé, le talc purifié et le stéarate de magnésium. Le résultat obtenu étant compressé dans une presse afin d'obtenir des comprimés d'un poids de 800 mg

Les comprimés obtenus sont alors recouverts par un film ayant les caractéristiques suivantes :

| Ingrédient | mg/ comprimé |
|---|---|
| Hydropropylmethylcellulose | 0,77 |
| Ph. Eur. 15 cps (Methocel E15) | |
| Hydroxypropylmethylcellulose | 3,87 |
| (Ph. Eur. 5 cps (Methocel E5) | |
| Opaspray M-1-7111B (33% solids) | 2,57 |
| Polyethylene glycol 400 USNF | 0,52 |
| Talc Purifié Ph. Eur. | 0,27 |
| Eau Purifiée Ph. Eur. | 55,52* |

| | |
|---|---|
| * Eliminée durant le processus | |

### Exemple 2 comprimé betaine fibrate

| Ingrédient | mg/ comprimé |
|---|---|
| Betaine Anhydre | 350,00 |
| Fenofibrate micronisé | 60,00 |
| (5 à 20 µm) | |
| Lactose Ph. Eur. | 35,00 |
| Ethylcellulose USNF | 90,00 |
| (Ethocel 45 CP) | |
| Eau pure Ph. Eur. | 120,00* |
| Alcool Cetostearyl | 32,00 |
| Ph. Eur. | |
| Magnesium Stearate | 17,00 |
| Ph. Eur. | |
| Talc Ph. Eur. | 16,00 |
| TOTAL | 600,00 |

| | |
|---|---|
| * Eliminée durant le processus | |

### Procédé d'obtention

La bétaine, préférablement de la glycine betaine monohydrate, le fenofibrate micronisé et le lactose ont été égrenés, puis transférés dans un granulateur vibrant et vaporisés avec de l'ethylcellulose et de l'eau. Les grains obtenus sont alors séchés à 60. degrés C. et passés par un tamis de 0,4 mm. Les granules obtenus sont alors mélangés avec l'alcool cetostearyl et le tout mixé énergiquement. La mixture obtenue est passée par un tamis de 0,5, laissée refroidir, puis est mixée avec le talc purifié et le stéarate de magnésium. Le résultat obtenu étant compressé dans une presse afin d'obtenir des comprimés d'un poids de 600 mg.

Les comprimés obtenus sont alors recouverts par un film ayant les caractéristiques de l'exemple 1.

### Exemple 3 comprimé betaine fibrate

On a répété les mêmes proportions que dans l'exemple 2, mais la bétaine et le fenofibrate ont été préalablement co-micronisés avec un agent tensioactif.

### Exemple 4 comprimé betaine niacin

| Ingrédient | mg/ comprimé |
|---|---|
| Niacine | 455,00 |
| Betaine | 285,00 |
| Hydroxypropyl Methylcellulose 2910 | 45,00 |
| (Methocel E15LV, Dow) | |
| Hydroxypropyl Methylcellulose 2208 | 98,00 |
| (Methocel K100 mCR, Dow) | |
| Huile végétale hydrogénée | 75,00 |
| (Lubritab, Mendell) | |
| Glyceryl Behenate 0.50 | 17,00 |
| (Compritol 888) | |
| Magnesium Stearate | 25,00 |
| TOTAL | 1000,00 |

### Procédé d'obtention

Pour réaliser le comprimé de l'eau purifiée est chauffée à 95 degrés C. dans un récipient en acier inoxydable. Le Methocel E15LV en poudre est lentement ajouté en mélangeant jusqu'à l'obtention d'une suspension homogène. La vitesse de rotation ayant été ajustée afin d'éviter l'entrée excessive d'air dans la solution par le vortex.. Puis de l'eau très froide est lentement ajoutée et le mélange mixé énergiquement jusqu'à ce qu'une solution claire ait été obtenue, ceci à une température inférieure à 20 degrés. C. Le mélange étant continué pendant 20 minutes supplémentaires.

L'huile végétale hydrogénée est passée alors à travers un tamis N° 16 et additionnée au mélangeur. Les poudres de niacin et de bétaine monohydrate sont alors ajoutées au mixeur et mélangées pendant à peu près 15 minutes, puis le Methocel K100 mCR est alors ajouté et le tout de nouveau mélangé pendant 15 minutes supplémentaires. Puis la solution de Methocel E15LV est vaporisée sur la mixture qui est encore mélangée pendant 2 minutes. Les granulés secs obtenus étant passés à travers un tamis N°16. Le résultat final étant compressé dans une presse excentrique afin de réaliser des comprimés de 1000 mg.

### Exemple 5 capsule betaine statine

### Préparation d'un granulé bétaine/statine à libération prolongée.

On effectue un mélange comprenant 37% d'un comprimable Eudragit RPL RTM, 61 % de bétaine anhydre, 1% de stéarate de talc et 1% magnésium. Après traitement dans un mélangeur Turbula T2C, le mélange a été compacté au moyen d'un appareil EKO de Korsch à 40,000 N/cm², puis a été transformé en granulés au moyen d'un granuleuse Erweka TG II S. Les granulés ont été ensuite triés au moyen d'une vibreuse ne gardant que les fractions ayant un diamètre entre 100 et 150. µm. Les granulés ainsi obtenus étant utilisés pour remplir des capsules multi-compartiments, dont un compartiment contient une statine (de 20 à 250 mg) destinée à être libérée immédiatement dans le tractus gastro-intestinal, et l'autre compartiment destiné à contenir le granulé de bétaine (de 400 à 1000 mg) apte à former une forme flottante dans l'estomac ou le début du tractus gastro-intestinal.

L'exemple 5 a été opéré avec l'atorvastatine, la cerivastatine, la cilastatine, la fluvastatine, la lovastatine, la nystatine, la pentostatine, la pravastatine, la simvastatine, et la somatostatine.

### Exemple 6

L'exemple 5 a été répété si ce n'est que la statine a été formulée ou une forme à libération contrôlée, par exemple grâce à une couche polyacrylate (méthacrylate) insoluble dans l'eau.

### Exemple 7

Les exemples 5 et 6 ont été répétés, si ce n'est qu'au lieu d'utiliser une statine, on a utilisé respectivement chacun des composés suivantes : befibrate, bezafibrate, ciprofibrate, clofibrate, clofibrate calcique, clofibrate d'aluminium, clofibrate de pyridoxine, clofibride, fenofibrate, fenofibrate micronisé, gemfibrozil, cholestyramine, le colesevelam, le colestipol, niacimide, l'inositol nicotinate, l'exaniacinate, le nicotinate sodique et l'acide nicotinique.

### Exemple 8

On a répété les exemples 1 et 2, si ce n'est qu'on a utilisé du fénofibrate co-micronisé en présence de glycine bétaine, le fénofibrate co-micronisé ayant une taille inférieure à 2 µm.

### Exemple 9

On a répété l'exemple 8, si ce n'est que le fénofibrate est nanonisé (réduit en particules de taille inférieure à 0,5µm, en particulier inférieure à 250 nm) en présence de glycine bétaine.

Les exemples ainsi décrits illustrent mais ne limitent pas l'invention.

## Revendications

1. Une composition pharmaceutique à usage oral comprenant du fénofibrate comicronisé avec de la bétaine, en particulier de la glycine betaine ou d'un sel de celle-ci.

2. Une composition pharmaceutique selon la revendication 1 dans laquelle le fénofibrate est comicronisé ou réduit en particules de moins de 1µm, en présence de bétaine, en particulier de glycine betaine ou d'un sel de celle-ci.

3. Une composition pharmaceutique selon là revendication 2 dans laquelle préférence au moins 50% du fénofibrate de la composition est comicronisé ou réduit en particules de moins de 1 µm en présence de bétaine.

4. Une composition suivant la revendication 1 **caractérisée en ce que** ladite composition contient en tant que surfactant ou tensioactif sensiblement uniquement de la bétaine, en particulier de la glycine bétaine, ladite bétaine formant plus de 90% en poids, de préférence plus de 95% en poids, plus spécifiquement plus de 99% en poids des surfactants ou tensioactifs présents dans la composition.

5. Une composition suivant la revendication 4 **caractérisée en ce que** le fénofibrate est mis sous formes micro et/ou nano sphéroïdiques et/ou sphéronisées en présence de bétaine et optionnellement en présence d'un ou plusieurs excipients,

6. Une composition suivant la revendication 5 **caractérisée en ce que** lesdits excipients peuvent optionnellement assurer une libération contrôlée d'une bétaine et/ou d'un agent anti-cholestérol, et/ou une biodisponibilité accrue.

7. Une composition suivant les revendications 4 à 6 **caractérisée en ce que** ladite composition est destinée améliorer la biodisponibilité du fénofibrate.

8. Un procédé de préparation de fibrate micronisé de taille moyenne de moins de 10 µm, en particulier de moins de 5 µm, de préférence de moins de 2 µm, préférentiellement de moins de 1 µm, dans lequel du fibrate solide est micronisé en présence de bétaine, le rapport en poids fibrate /betaine étant avantageusement inférieur à 1.

## Claims

1. A pharmaceutical composition for oral use comprising fenofibrate co-micronised with betaine, in particular glycine betaine or a salt thereof.

2. A pharmaceutical composition according to claim 1 in which the fenofibrate is co-micronised or reduced in particles of less than 1 µm, in presence of betaine, in particular glycine betaine or a salt thereof.

3. A pharmaceutical composition according to claim 2 in which preferably at least 50% of the fenofibrate of the composition is co-micronised or reduced in particles of less than 1 µm in presence of betaine.

4. A composition according to claim 1 **characterised in that** said composition contains as surfactant or tensioactive sensibly uniquely betaine, in particular glycine betaine, said betaine forming more than 90% in weight, preferably more than 95% in weight, more specifically more than 99% in weight of the surfactants or tensioactives present in the composition.

5. A composition according to claim 4 **characterised in that** the fenofibrate is put in micro and/or nano spheroid and/or spheronised forms in presence of betaine and optionally in presence of one or more excipients.

6. A composition according to claim 5 **characterised in that** said excipients can optionally assure a controlled release of a betaine and/or of an anti-cholesterol agent, and/or an increased bioavailability.

7. A composition according to claims 4 to 6 **characterised in that** said composition is destined to ameliorate fenofibrate bioavailability.

8. A preparation process of micronised fenofibrate of mean size of less than 10 µm, in particular of less than 5 µm, preferably of less than 2 µm, more preferably of less than 1 µm, in which solid fenofibrate is micronised in presence of betaine, the weight ratio fibrate/betaine being advantageously inferior to 1.

## Patentansprüche

1. Pharmazeutische Zusammensetzung zur oralen Verabreichung, die Fenofibrat comikronisiert mit Betain enthält, insbesondere mit Glycinbetain oder einem Salz desselben.

2. Pharmazeutische Zusammensetzung nach Anspruch 1, in welcher das Fenofibrat in Gegenwart von Betain, insbesondere von Glycinbetain oder eines Salzes desselben, zu Partikeln von weniger als 1 µm comikronisiert oder zerkleinert ist.

3. Pharmazeutische Zusammensetzung nach Anspruch 2, in welcher vorzugsweise mindestens 50 % des Fenofibrats der Zusammensetzung in Gegenwart von Betain zu Partikeln von weniger als 1 µm comikronisiert oder zerkleinert sind.

4. Zusammensetzung nach Anspruch 1, **dadurch gekennzeichnet, dass** die Zusammensetzung als oberflächenaktives Mittel oder Tensid im Wesentlichen ausschließlich Betain enthält, insbesondere Glycinbetain, wobei das Betain mehr als 90 Gew.-%, vorzugsweise mehr als 95 Gew.-%, noch spezieller mehr als 99 Gew.-% der in der Zusammensetzung vorhandenen oberflächenaktiven Mittel oder Tenside ausmacht.

5. Zusammensetzung nach Anspruch 4, **dadurch gekennzeichnet, dass** das Fenofibrat in Gegenwart von Betain und optional in Gegenwart eines oder mehrerer Exzipienten in mikrosphäroidale und/oder nanosphäroidale und/oder sphäronisierte Formen gebracht wird.

6. Zusammensetzung nach Anspruch 5, **dadurch gekennzeichnet, dass** die Exzipienten optional eine kontrollierte Freisetzung eines Betains und/oder eines Anticholesterinmittels, und/oder eine erhöhte Bioverfügbarkeit sicherstellen können.

7. Zusammensetzung nach den Ansprüchen 4 bis 6, **dadurch gekennzeichnet, dass** die Zusammensetzung dazu bestimmt ist, die Bioverfügbarkeit des Fenofibrats zu verbessern.

8. Verfahren zur Herstellung von mikronisiertem Fibrat mit einer mittleren Größe von weniger als 10 µm, insbesondere von weniger als 5 µm, vorzugsweise von weniger als 2 µm, noch mehr bevorzugt von weniger als 1 µm, bei welchem festes Fibrat in Gegenwart von Betain mikronisiert wird, wobei das Gewichtverhältnis Fibrat/Betain vorteilhafterweise kleiner als 1 ist.
